# EUROPEAN PATENT APPLICATION

(11) **EP 4 692 777 A1**
(43) Date of publication of application: **11.02.2026**
(21) Application number: 24776824.5
(22) Date of filing: 21.03.2024
(51) Int. Cl.: G01N 27/62, G01N 33/50

(54) **INFORMATION PROCESSING DEVICE, INFORMATION PROCESSING METHOD, AND COMPUTER PROGRAM**

(30) Priority: 30.03.2023 JP 2023054539
(71) Applicant: National University Corporation Tokai National Higher Education and Research System, Nagoya-shi, Aichi 464-8601 (JP)
(72) Inventor: BABA Taisuke, Nagoya-shi, Aichi 464-8601 (JP); SUNAGAWA Masaki, Nagoya-shi, Aichi 464-8601 (JP); KOKURYO Toshio, Nagoya-shi, Aichi 464-8601 (JP); MIZUNO Takashi, Nagoya-shi, Aichi 464-8601 (JP)
(74) Representative: Grünecker Patent- und Rechtsanwälte PartG mbB
(86) International application number: PCT/JP2024/010963
(87) International publication number: WO 2024/203701

(57) **Abstract**

An information processing device includes a model acquisition unit, an object data acquisition unit, and a classification execution unit. The model acquisition unit acquires a learned model taking comparison data as input and outputting a classification result of a sample. In the mass spectrometry result for the plurality of the substances contained in the sample, for at least one substance group pair, which is the pair of the two substance groups each containing at least one substance, the comparison data is the data indicating the magnitude relationship between the peak intensity of the substance belonging to the one substance group and the peak intensity of the substance belonging to the other substance group. The object data acquisition unit acquires the comparison data on an object sample. The classification execution unit inputs the comparison data on the object sample to the learned model, thereby classifying the object sample, and outputting the classification result.

## Description

### TECHNICAL FIELD

A technique disclosed in the present specification relates to information processing for sample classification.

### BACKGROUND ART

Various markers have been used to diagnose diseases. For example, CA19-9 is used as a tumor marker for pancreatic ductal carcinoma and bile duct carcinoma (for example, see Patent Literature 1).

### CITATION LIST

### Patent Literature

Patent Literature 1: Japanese Unexamined Patent Application Publication No. 2023-37003

### SUMMARY OF INVENTION

### TECHNICAL PROBLEM

It is considered to classify whether a person to be diagnosed suffers from a specific disease with reference to a peak area of a specific substance acquired by subjecting a sample (blood, urine, or the like) collected from the person to be diagnosed to a mass spectrometry. However, a result of the mass spectrometry can vary due to various factors. Thus, a standard material is used in a quantitative mass spectrometry of the specific substance. However, in such a quantitative mass spectrometry, content of substances other than the specific substance measured at the same time is not used for the diagnosis, and there is room for improvement in diagnostic accuracy. Such a problem is not limited to a case where a purpose is to diagnose the specific disease, but is common to a case where any form of classification is performed with reference to the mass spectrometry result of the sample.

The present specification discloses a technique capable of solving the above-described problem.

### SOLUTION TO PROBLEM

The technique disclosed in the present specification can be implemented, for example, as the following aspects.
(1) An information processing device disclosed herein includes a model acquisition unit, an object data acquisition unit, and a classification execution unit. The model acquisition unit acquires a learned model taking comparison data as input and outputting a classification result of a sample. In a mass spectrometry result for a plurality of substances contained in the sample, for at least one substance group pair, which is a pair of two substance groups each containing at least one substance, the comparison data contains data indicating a magnitude relationship between a peak intensity of the substance belonging to one of the substance groups and a peak intensity of the substance belonging to the other substance group. The object data acquisition unit acquires the comparison data on an object sample. The classification execution unit inputs the comparison data on the object sample to the learned model, thereby classifying the object sample, and outputting a result of the classification.
   As described above, in this information processing device, the object sample is classified by using the learned model which takes the comparison data as the input and outputs the sample classification result. In the mass spectrometry result for the plurality of the substances contained in the sample, for at least one substance group pair, which is the pair of the two substance groups each containing at least one substance, the comparison data contains data indicating the magnitude relationship between the peak intensity of the substance belonging to the one substance group and the peak intensity of the substance belonging to the other substance group. Accordingly, by using the comparison data, it is possible to relatively reduce an influence of a variation in a value between the samples caused by various factors and to improve accuracy of the classification of the sample with reference to the mass spectrometry result. Since the comparison data is the data created by using mass spectrometry results of the plurality of the substances contained in the sample, it is possible to improve the accuracy of the classification of the sample with reference to the mass spectrometry results in comparison with a configuration that only uses a mass spectrometry result of a specific substance.
(2) The information processing device described above may be configured that the comparison data contains data indicating a ratio of the peak intensity of the substance belonging to the one substance group to the peak intensity of the substance belonging to the other substance group. By adopting this configuration, the substance group pair can be formed by any combination of the substances, without being limited to a pair of the substances whose peak intensities in the sample are substantially the same and whose magnitude relationship is inverted depending on the belonging classification. As a result, a degree of freedom in setting of the substance group pair is increased. Thus, the further preferable comparison data can be acquired, and the accuracy of the classification of the sample with reference to the mass spectrometry result can be effectively improved.
(3) The information processing device described above may be configured that the comparison data contains data indicating a ratio of the peak intensity of each of the substances belonging to the one substance group to an average value of the peak intensities of all the substances belonging to the other substance group. By adopting this configuration, the value of the comparison data is determined for each of the substances, and a reduction in an amount of information can be suppressed. As a result, the comparison data having a larger amount of the information can be acquired, and the accuracy of the classification of the sample with reference to the mass spectrometry result can be effectively improved.
(4) The information processing device described above may be configured that the comparison data contains data on a plurality of the substance group pairs. By adopting this configuration, the comparison data having the larger amount of the information can be acquired, and the accuracy of the classification of the sample with reference to the mass spectrometry result can be effectively improved.
(5) The information processing device described above may be configured to further include a pair setting unit that sets the at least one substance group pair based on correlation between the plurality of the substances. By adopting this configuration, it is possible to set the substance group pair that contributes to the further appropriate classification, and to effectively improve the accuracy of the classification of the sample with reference to the mass spectrometry result.
(6) The information processing device described above may be configured that the sample is a biological sample derived from a human. By adopting this configuration, it is possible to improve accuracy of classification of the biological sample with reference to a mass spectrometry result of the biological sample.
(7) The information processing device described above may be configured that the biological sample is at least one of urine, bile, sweat, saliva, blood, cerebrospinal fluid (CSF), ascites, pleural fluid, pancreatic juice, gastric juice, and intestinal juice. By adopting this configuration, it is possible to improve the accuracy of the classification of the biological sample with reference to the mass spectrometry results of those biological samples.
(8) The information processing device described above may be configured that the classification execution unit executes the classification for a specific disease. By adopting this configuration, it is possible to improve accuracy of a diagnosis of the specific disease with reference to the mass spectrometry result.
(9) The information processing device described above may be configured that the specific disease is cancer. By adopting this configuration, it is possible to improve accuracy of a diagnosis of cancer with reference to the mass spectrometry result.

The technique disclosed in the present specification can be achieved in various forms, and for example, can be achieved in the form of the information processing device, an information processing method, a computer program that implements those methods, a non-transitory recording medium recording the computer program, or the like.

### BRIEF DESCRIPTION OF DRAWINGS

FIG. 1 is an explanatory view schematically showing a diagnostic model MO in the present embodiment.
FIG. 2 is an explanatory view schematically showing the diagnostic model MO in the present embodiment.
FIG. 3 is an explanatory view conceptually showing mass spectrometry data MD and comparison data CD.
FIG. 4 is an explanatory diagram showing a schematic configuration of an information processing device 100.
FIG. 5 is a flowchart showing diagnostic model acquisition processing in the present embodiment.
FIG. 6 is a flowchart showing diagnostic processing in the present embodiment.
FIG. 7 is an explanatory chart showing a principal component analysis result of the mass spectrometry data MD.
FIG. 8 is an explanatory chart showing a principal component analysis result of the mass spectrometry data MD narrowed down to a cancer-specific substance.
FIG. 9 is an explanatory chart showing a principal component analysis result of the comparison data CD.
FIG. 10 is an explanatory chart showing accuracy of the diagnostic model MO in the present embodiment.
FIG. 11 is an explanatory chart showing classification accuracy in a case where the diagnostic model MO in the present embodiment is applied to data on pancreatic cancer.
FIG. 12 includes explanatory charts showing the principal component analysis result and the classification accuracy in a case where the classification is performed using creatinine correction.
FIG. 13 includes explanatory charts showing the principal component analysis result and the classification accuracy in a case where the classification is performed using the diagnostic model MP in the present embodiment.

### EMBODIMENTS OF THE INVENTION

### A. Embodiment:

### A-1. Overview of Diagnostic Model MO:

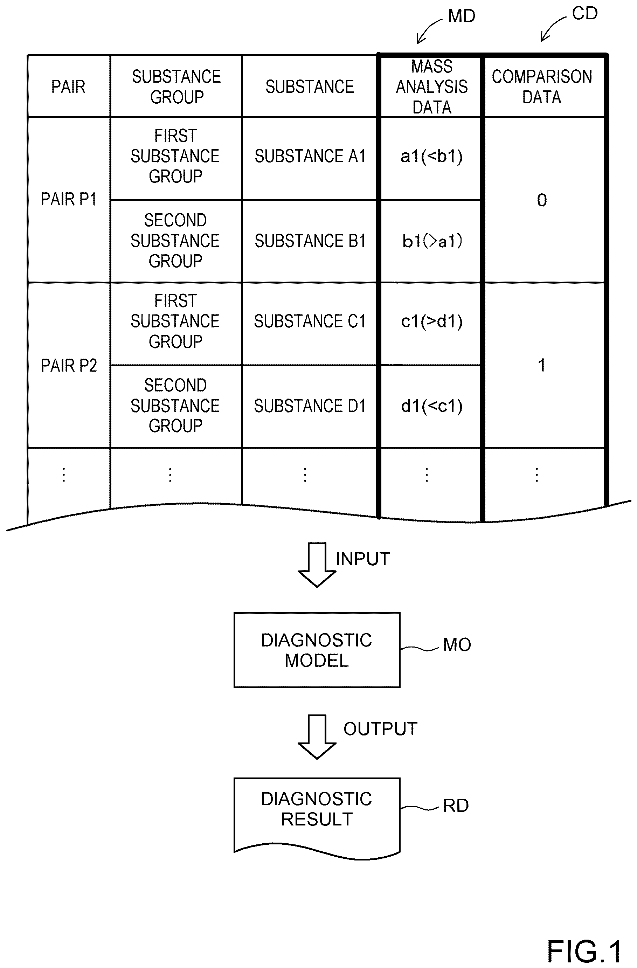

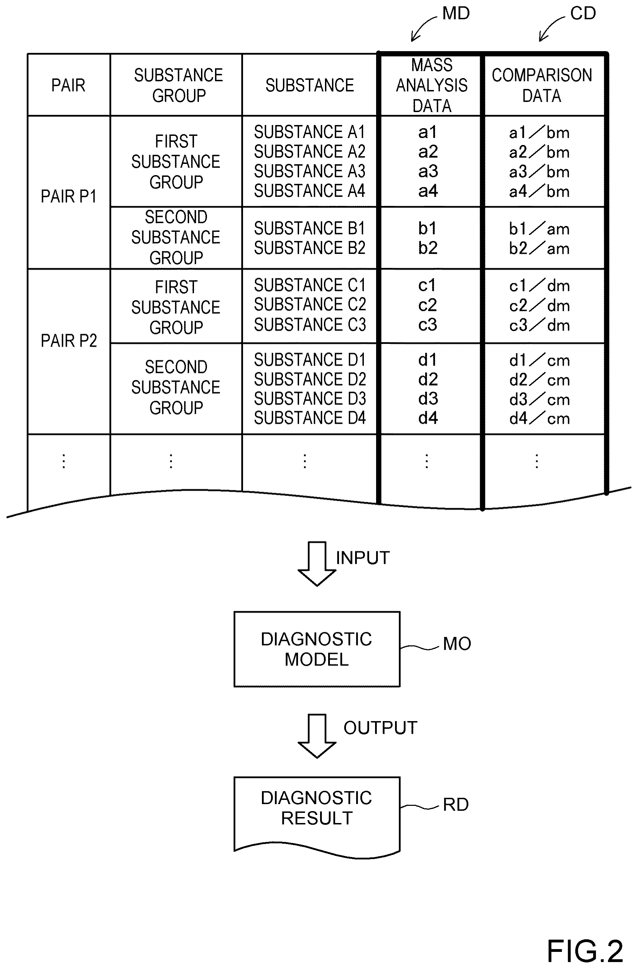

FIG. 1 and FIG. 2 are explanatory views schematically showing a diagnostic model MO in the present embodiment. The diagnostic model MO is a learned model for diagnosing a specific disease, and is used to classify whether a person to be diagnosed suffers from the specific disease, for example. Examples of the specific disease include cancer (biliary tract cancer, pancreatic cancer, gastric cancer, colorectal cancer, and the like) and diseases other than cancer (an inflammatory bowel disease and the like). In the following description, it is assumed that the specific disease is cancer (more specifically, biliary tract cancer).

As shown in FIG. 1 and FIG. 2, input to the diagnostic model MO is comparison data CD that is specified based on mass spectrometry data MD of a biological sample collected from the person to be diagnosed as a subject. As the biological sample, for example, urine, bile, sweat, saliva, blood, cerebrospinal fluid (CSF), ascites, pleural fluid, pancreatic juice, gastric juice, intestinal juice, or the like is used. In the following description, it is assumed that the biological sample is urine, and objects of a mass spectrometry are a plurality of substances (metabolites (metabolome)) contained in urine. In the mass spectrometry of urine as the object, for example, peak areas (peak intensities) of a large number of the substances that are about 1500 to 2000 are measured.

In the mass spectrometry, a result thereof can vary due to various factors, such as sample collection conditions, sample processing conditions prior to the analysis, daily setups of an analytical instrument (a batch to batch error), and a substance present in the sample that interferes with ionization (a matrix effect). The biological sample such as urine has a variation in concentration, and a variation in the analysis result is likely to be relatively large in the mass spectrometry of the biological sample as the object. Thus, in the present embodiment, as will be described below, the comparison data CD that is specified based on the mass spectrometry data MD indicating the mass spectrometry result is used.

As shown in FIG. 1 and FIG. 2, in order to generate the comparison data CD, a plurality of substance group pairs (hereinafter, also simply referred to as "pairs") is set in advance for the plurality of the substances contained in the sample. The substance group pair is a pair of two substance groups (a first substance group and a second substance group), each of which contains at least one substance. In an example shown in FIG. 1, both of the two substance groups constituting the respective substance group pair contain a single substance. For example, a first substance group pair P1 is a pair of the first substance group containing a single substance A1 and the second substance group containing another single substance B1. Just as described, in the present specification, a unit composed of a single element is also referred to as a "group".

In an example shown in FIG. 2, the two substance groups constituting the respective substance group pair contain one or plural substances. For example, the first substance group pair P1 is a pair of the first substance group containing four substances A1, A2, A3, A4 and the second substance group containing two substances B1, B2.

The plurality of the substance group pairs is set based on correlation between the plurality of the substances contained in the sample. This point will be described in detail below.

The comparison data CD for each of the substance group pairs contains data that indicates a magnitude relationship between the peak area of the substance belonging to one substance group (the first substance group or the second substance group) and the peak area of the substance belonging to the other substance group (the second substance group or the first substance group). For example, in the example of FIG. 1, the comparison data CD for each of the substance group pairs is binary data of "0/1" which takes a value "0" when the peak area of the substance belonging to the first substance group is smaller than the peak area of the substance belonging to the second substance group, and takes a value "1" when the peak area of the substance belonging to the first substance group is greater than or equal to the peak area of the substance belonging to the second substance group. For example, for the first substance group pair P1, since a peak area a1 of the substance A1 constituting the first substance group is smaller than the peak area b1 of the substance B1 constituting the second material group, the comparison data CD takes the value "0". For a second substance group pair P2, since a peak areas c1 of a substance C1 constituting the first substance group is greater than or equal to a peak area d1 of a substance D1 constituting the second substance group, the comparison data CD takes the value "1".

The comparison data CD for each of the substance group pairs may contain data indicating a ratio of the peak area of the substance belonging to one substance group to the peak area of the substance belonging to the other substance group. For example, in the example of FIG. 2, the comparison data CD for each of the substances belonging to one substance group constituting the respective substance group pair is a value acquired by dividing the peak area of each of the substances belonging to the one substance group by an average value of the peak areas of all the substances belonging to the other substance group. For example, for the substance A1 belonging to the first substance group of the first substance group pair P1, it is a value acquired by dividing the peak area a1 of the substance A1 by an average value bm (= (b1 + b2)/2) of the peak areas of all the substances (the substances B1, B2) belonging to the second substance group of the pair. For example, for the substance B1 belonging to the second substance group of the pair, it is a value acquired by dividing the peak area b1 of the substance B1 by an average value am (= (a1 + a2 + a3 + a4)/4) of the peak areas of all the substances (the substances A1, A2, A3, A4) belonging to the first substance group of the pair. In each of the substance group pairs, in a case where the value of the comparison data CD calculated for the substance belonging to one substance group is less than 1, it can be said that the peak area of the substance belonging to such a substance group is smaller than the peak area of the substance belonging to the other substance group. On the contrary, in a case where the value of the comparison data CD calculated for the substance belonging to the one substance group is greater than or equal to 1, it can be said that the peak area of the substance belonging to such a substance group is greater than or equal to the peak area of the substance belonging to the other substance group. Accordingly, also in the example of FIG. 2, it can be said that the comparison data CD for each of the substance group pairs is the data indicating the magnitude relationship between the peak area of the substance belonging to one substance group and the peak area of the substance belonging to the other substance group.

Just as described, the comparison data CD for each of the substance group pairs is information indicating which of the substance belonging to one substance group and the substance belonging to the other substance group has the larger peak area. This comparison data CD may contain information indicating which of the substances belonging to one substance group and the substances belonging to the other substance group has the larger peak area and how large the peak area is. By using such comparison data CD, it is possible to relatively reduce an influence of a variation in the value between the samples due to various factors.

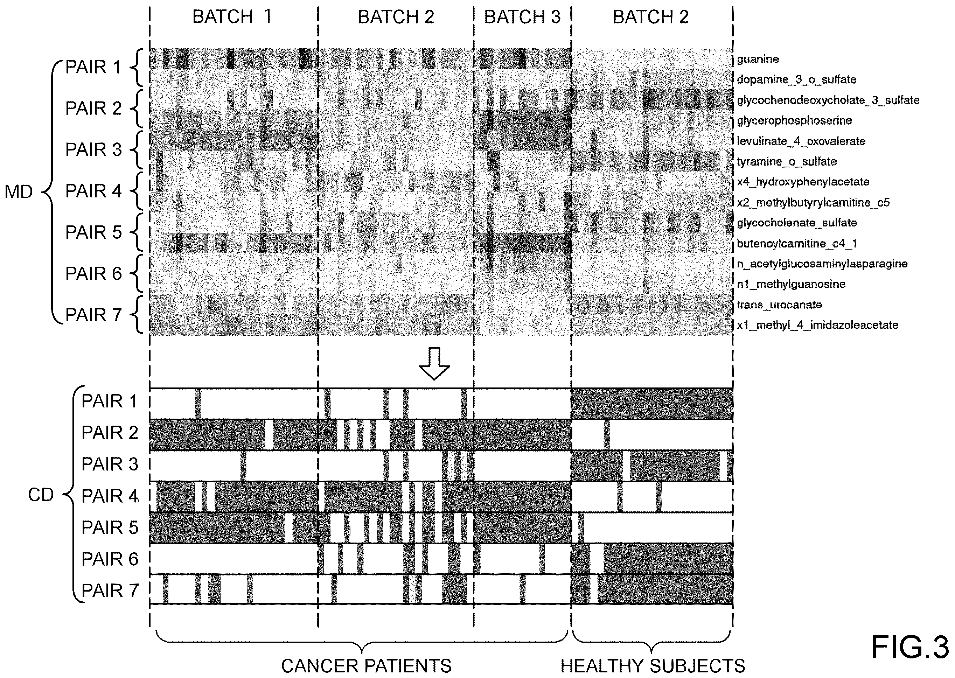

FIG. 3 is an explanatory view conceptually showing the mass spectrometry data MD and the comparison data CD. In FIG. 3, "BATCH 1", "BATCH 2", and "BATCH 3" each represents a sample collection unit, and different batches can have the different sample collection conditions. Right "BATCH 2" of two is data on the sample collected from a healthy subject, and the remaining one is data on the sample collected from a biliary tract cancer patient.

An upper portion of FIG. 3 conceptually shows an example of the mass spectrometry data MD. The mass spectrometry data MD is data indicating the peak areas of the plurality of the substances (for example, guanine, dopamine_3_o_sulfate, glycochenodeoxycholate_3_sulfate, and the like) that is contained in the sample and is specified by the mass spectrometry of the sample. In FIG. 3, the peak area of each of the substances is represented by concentration. As shown in the drawing, even for the samples collected from the biliary tract cancer patients, a tendency of the peak area of each of the substances in urine can significantly vary only by differences in measurement dates of the mass spectrometry.

A lower portion of FIG. 3 conceptually shows an example of the comparison data CD. In an example shown in FIG. 3, similar to the example shown in FIG. 1, the two substance groups constituting the respective substance group pair each contain the single substance, and the comparison data CD for each of the substance group pairs is the binary data of "0/1" which takes the value "0" when the peak area of the substance belonging to the first substance group is smaller than the peak area of the substance belonging to the second substance group. On the contrary, it takes the value "1" when the peak area of the substance belonging to the first substance group is greater than or equal to the peak area of the substance belonging to the second substance group. For the biliary tract cancer patients, the tendencies of the comparison data CD of each of the substance group pairs are similar to each other regardless of the batch, and are significantly different from the tendencies of the comparison data CD for the healthy subjects. Just as described, by using the comparison data CD for each of the substance group pairs, it is possible to relatively reduce the influence of the variation in the value between the samples due to the various factors.

As shown in FIG. 1 and FIG. 2, output of the diagnostic model MO is diagnostic result data RD indicating a diagnostic result. The diagnostic result is a classification result of an object sample and, more specifically, is a classification result of whether the person to be diagnosed who is a collection source of urine as the object sample suffers from biliary tract cancer.

As described so far, in the mass spectrometry result for the plurality of the substances contained in the sample, for the substance group pair, which is the pair of the two substance groups each containing at least one substance, the comparison data CD as the input contains the data indicating the magnitude relationship between the peak area of the substance belonging to one substance group and the peak area of the substance belonging to the other substance group, and the diagnostic model MO as the learned model having the classification result of the sample as the output is used. In this way, it is possible to relatively reduce the influence of the variation in the value between the samples due to the various factors and to achieve the classification of the sample with a higher degree of accuracy, that is, the diagnosis of the disease with a higher degree of accuracy. The classification method in the present embodiment is also referred to as Inverse Pair Boosting (abbreviated as "IPB").

### A-2. Configuration of Information Processing Device:

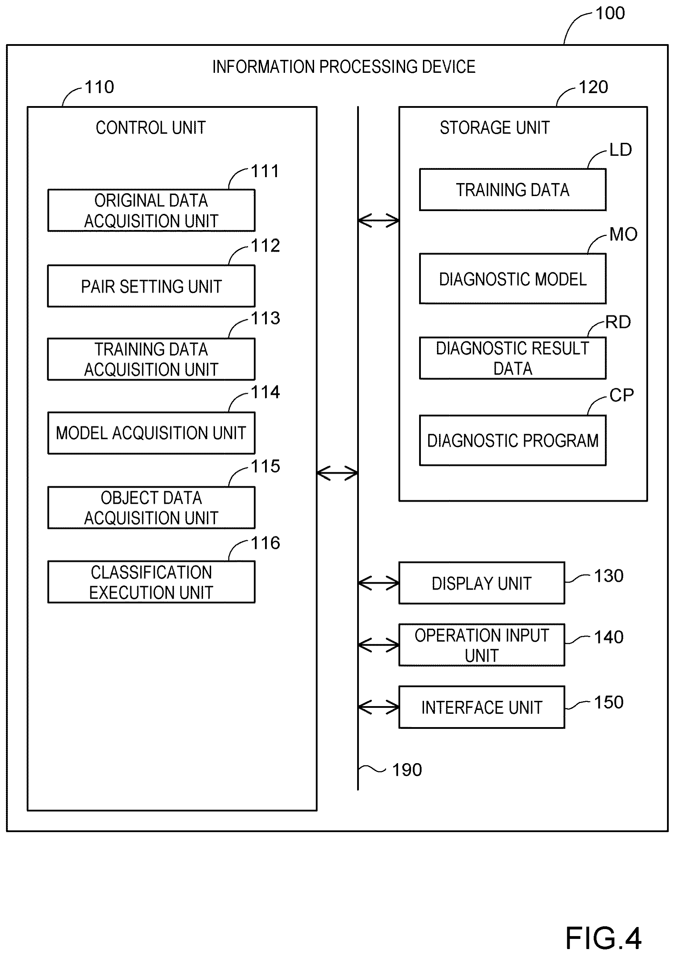

Next, a configuration of an information processing device 100 for creating the diagnostic model MO and executing a diagnosis using the diagnostic model MO will be described. FIG. 4 is an explanatory diagram showing a schematic configuration of the information processing device 100. The information processing device 100 is formed by a computer (a personal computer (PC), a server, and the like).

The information processing device 100 includes a control unit 110, a storage unit 120, a display unit 130, an operation input unit 140, and an interface unit 150. These units are communicably connected to each other via a bus 190. The information processing device 100 may include a speaker as an output unit.

The display unit 130 in the information processing device 100 includes, for example, a liquid-crystal display or the like, and displays various images and information. The operation input unit 140 includes, for example, a keyboard, a mouse, a button, a microphone, a track pad, and the like, and receives an operation or an instruction from an administrator. The display unit 130 may include a touch panel to function as the operation input unit 140. The interface unit 150 includes, for example, a LAN interface, a USB interface, or the like, and performs wired or wireless communication with other devices.

The storage unit 120 in the information processing device 100 includes, for example, read only memory (ROM), random access memory (RAM), a hard disk drive (HDD), and the like, stores various programs and data, and is used as a work area when the various programs are executed or as a temporary storage area of the data. For example, the storage unit 120 stores a diagnostic program CP as a computer program for executing diagnostic model acquisition processing or diagnostic processing described below. The diagnostic program CP is provided in a state of being stored in a computer-readable recording medium (not shown), such as a CD-ROM, a DVD-ROM, or USB memory, or is provided in a state of being acquirable from an external device (a server on a network or another terminal device) via the interface unit 150 and is stored in the storage unit 120 in a state of being operable on the information processing device 100.

In the storage unit 120 of the information processing device 100, training data LD, the diagnostic model MO, and the diagnostic result data RD are stored in advance or during various types of processing described below. Contents of these pieces of information and data will be described together with the description of the various types of processing described below.

The control unit 110 in the information processing device 100 includes, for example, a central processing unit (CPU) and the like, and controls the operation of the information processing device 100 by executing the computer program read from the storage unit 120. For example, the control unit 110 reads and executes the diagnostic program CP from the storage unit 120 to execute the various types of processing described below. At this time, the control unit 110 functions as an original data acquisition unit 111, a pair setting unit 112, a training data acquisition unit 113, a model acquisition unit 114, an object data acquisition unit 115, and a classification execution unit 116. The functions of these units will be described together with the description of the various types of processing described below.

### A-3. Diagnostic Model Acquisition Processing:

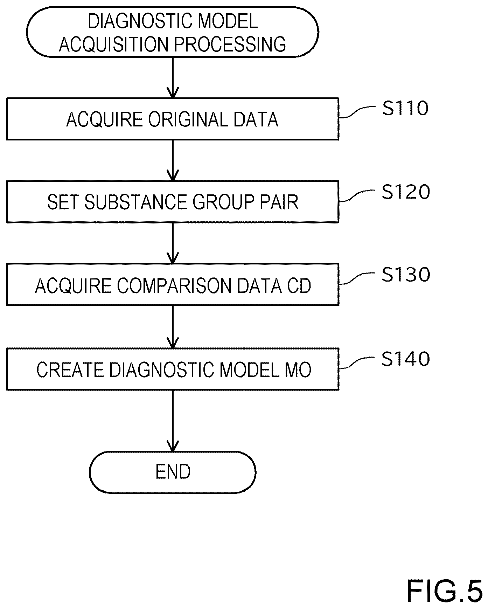

Next, the diagnostic model acquisition processing executed by the information processing device 100 in the present embodiment will be described. FIG. 5 is a flowchart showing the diagnostic model acquisition processing in the present embodiment. The diagnostic model acquisition processing is processing for acquiring the diagnostic model MO described above. In the present embodiment, the information processing device 100 creates the diagnostic model MO by predetermined machine learning by itself and thereby acquires the diagnostic model MO. The diagnostic model acquisition processing is started in response to input of a start instruction by a user operating the operation input unit 140 of the information processing device 100.

First, the original data acquisition unit 111 (FIG. 4) in the information processing device 100 acquires original data that is used to create the diagnostic model MO (S110). The original data is the mass spectrometry data MD (see FIGs. 1 and 2) indicating the mass spectrometry result of urine as the biological samples collected from a plurality of the biliary tract cancer patients and healthy subjects (controls). In the original data, the peak areas of the large number of the substances (metabolites) contained in urine are specified. The original data is acquired via the interface unit 150 or the operation input unit 140.

Next, the pair setting unit 112 (FIG. 4) in the information processing device 100 sets the plurality of the substance group pairs (S120). As described above, the substance group pair is a pair of the two substance groups each containing at least one substance (see FIGs. 1 and 2). The pair setting unit 112 sets the plurality of the substance group pairs based on the correlation between the plurality of the substances contained in the sample.

A specific setting method of the plurality of the substance group pairs by the pair setting unit 112 is, for example, as follows. First, Wilcoxon rank-sum test is performed on the substances (metabolites) outside 95% of the healthy subjects, for example, and the substances with a sufficiently large statistically significant differences are selected. It can be said that the selected substance is a cancer-specific substance. Among the selected substances, peak area data of the substance which is reduced as compared with the healthy subjects (which satisfies a relationship of Log2 fold change (L2FC) < 0) is multiplied by -1 to invert a sign, and the Pearson's coefficient of correlation is calculated. Unsupervised clustering (for example, using M3C of an R package) using this coefficient of correlation as a distance is performed up to maxK = 10, and the plurality of the substances are classified into the number of clusters having the lowest p value. By clustering together the data whose signs have been inverted as described above, substance groups that exhibit inverse correlation are grouped into the same cluster. These inversely correlated substance groups in the same cluster are paired. It is determined whether there are sufficient (for example, 5 or more) substances showing the inverse correlation in the cluster, and the cluster having the insufficient substances is paired with another cluster having the close Pearson's coefficient of correlation. Each of the clusters set as described above becomes the substance group pair. In cancer, abnormalities occur in various metabolic pathways due to metabolic reprogramming. When the metabolism does not proceed at a certain point in the metabolic pathway, the substance groups before the metabolism are increased, and conversely, the substance groups after the metabolism are reduced. By exhaustively extracting the thus-generated inverse correlations among the plurality of the substance groups on the metabolic pathway, it is possible to set the substance group pair including the two substance groups that highlight the differences in metabolism between the biliary tract cancer patients and the healthy subjects.

Next, the training data acquisition unit 113 (FIG. 4) in the information processing device 100 acquires the above-described comparison data CD on the plurality of the set substance group pairs (S130). For example, as shown in FIG. 1, the training data acquisition unit 113 acquires, as the comparison data CD on the plurality of the substance group pairs, the binary data of "0/1" which takes the value "0" when the peak area of the substance belonging to the first substance group is smaller than the peak area of the substance belonging to the second substance group, and takes the value "1" when the peak area of the substance belonging to the first substance group is greater than or equal to the peak area of the substance belonging to the second substance group. Alternatively, as shown in FIG. 2, the training data acquisition unit 113 calculates, as the comparison data CD on the plurality of the substance group pairs, the value calculated by dividing the peak area of each of the substances belonging to the one substance group by the average value of the peak areas of all the substances belonging to the other substance group. The training data acquisition unit 113 creates the training data LD by using the acquired comparison data CD. The training data LD is data in which the comparison data CD is associated with distinctions between the biliary tract cancer patients and the healthy subjects.

Next, the model acquisition unit 114 (FIG. 4) in the information processing device 100 creates the diagnostic model MO by the machine learning using the training data LD (S140). Any of various known machine learning algorithms can be used for the machine learning to create the diagnostic model MO. For example, a random forest or a support vector machine may be used to create the diagnostic model MO. The diagnostic model MO created by the machine learning is stored in the storage unit 120 of the information processing device 100. As described so far, the acquisition processing of the diagnostic model MO (FIG. 5) is completed.

### A-4. Diagnostic Processing:

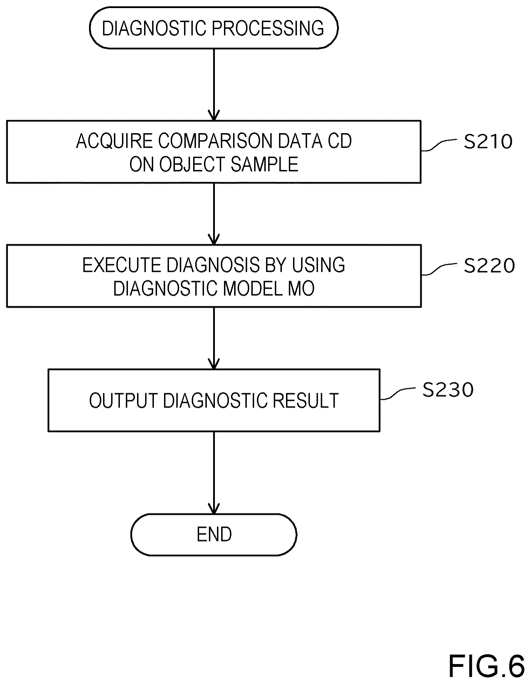

Next, the diagnostic processing executed by the information processing device 100 in the present embodiment will be described. FIG. 6 is a flowchart showing the diagnostic processing in the present embodiment. The diagnostic processing is processing to classify the object sample by using the diagnostic model MO, more specifically, to classify whether the person to be diagnosed as the collection source of the object sample suffers from biliary tract cancer. The diagnostic processing is started in response to the input of the start instruction by the user operating the operation input unit 140 of the information processing device 100.

First, the object data acquisition unit 115 (FIG. 4) in the information processing device 100 acquires the comparison data CD on the object sample (S210). The object sample is urine collected from the person to be diagnosed. As described above, the comparison data CD contains, for each of the substance group pairs: the data that indicates the magnitude relationship between the peak area of the substance belonging to one substance group and the peak area of the substance belonging to the other substance group; or the data that indicates the ratio of the peak area of the substance belonging to one substance group to the peak area of the substance belonging to the other substance group. The object data acquisition unit 115 acquires the mass spectrometry data MD of the object sample via the interface unit 150, and creates the comparison data CD based on the mass spectrometry data MD. Alternatively, the object data acquisition unit 115 acquires the comparison data CD, which is created in advance based on the mass spectrometry data MD, via the interface unit 150.

Next, the classification execution unit 116 (FIG. 4) in the information processing device 100 executes the diagnosis by using the comparison data CD of the object sample and the diagnostic model MO (S220). More specifically, the classification execution unit 116 inputs the comparison data CD on the object sample to the diagnostic model MO, and thereby acquires the classification result (the classification result of whether the subject suffers from biliary tract cancer) output from the diagnostic model MO. The classification execution unit 116 generates the diagnostic result data RD indicating the diagnostic result, and stores the diagnostic result data RD in the storage unit 120 of the information processing device 100.

Next, the classification execution unit 116 outputs the diagnostic result based on the diagnostic result data RD (S230). For example, the classification execution unit 116 causes the display unit 130 to display the diagnostic result. As described so far, the diagnostic processing is completed.

### A-5. Example:

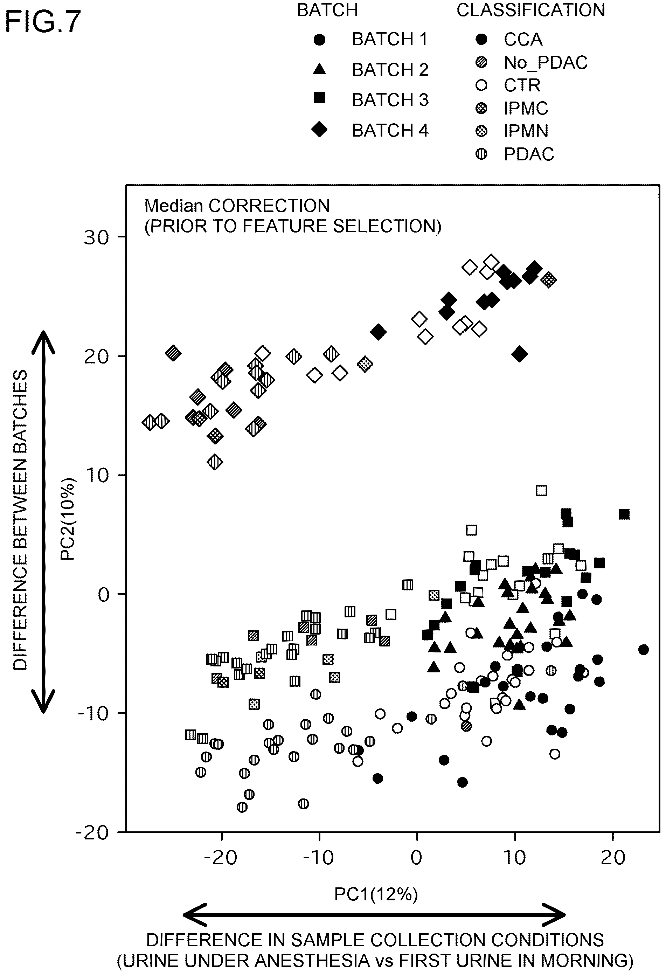

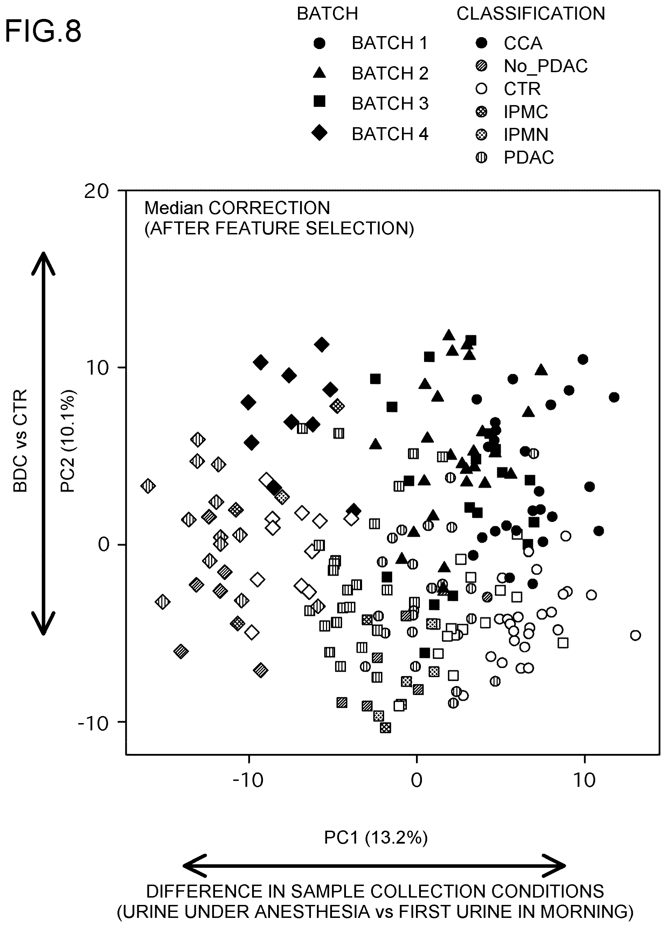

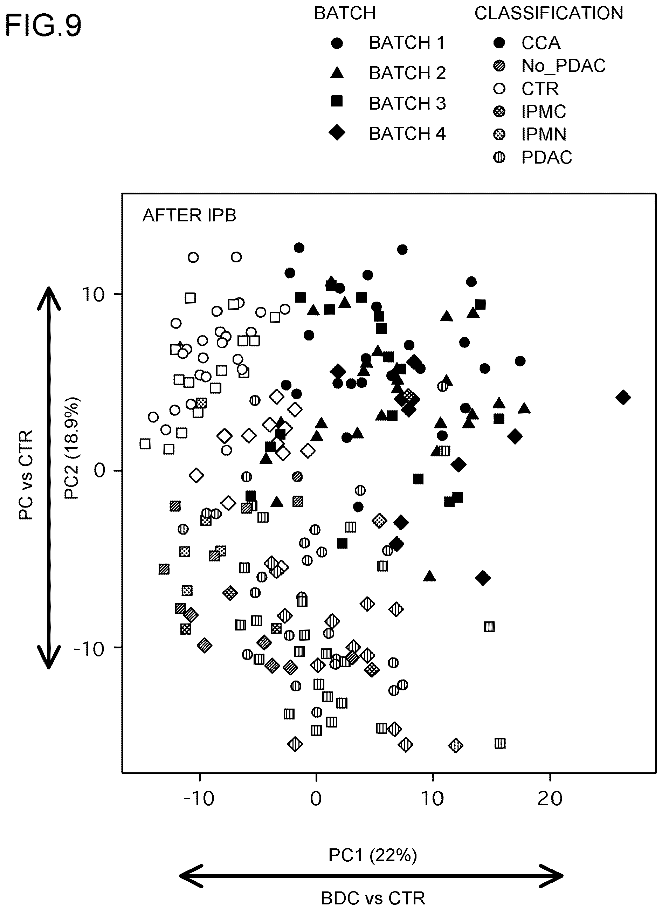

An example of the diagnostic model MO described above will be described below. FIG. 7 is an explanatory chart showing a principal component analysis result of the mass spectrometry data MD. In FIG. 7, an X-axis represents a first principal component (PC1), and a Y-axis represents a second principal component (PC2). Each of the axes shows a contribution rate of each of the components. In FIG. 7, the classification of the object data is indicated by hatching of each plot. In the drawing, "CCA" is a sample of the biliary tract cancer patient, "No_PDAC" is a sample of a case where a person is suspected to have pancreatic cancer and operated but is found not to have pancreatic cancer by a pathological diagnosis, "CTR" is a sample of the healthy subject (control), "IPMC" is a sample of a kind of Intraductal Papillary Mucinous Carcinoma precancerous lesion (after canceration), "IPMN" is a sample of a kind of Intraductal Papillary Mucinous Neoplasm precancerous lesion (before the canceration), and "PDAC" is a sample of a pancreatic cancer patient. In FIG. 7, a shape of each of the plots indicates the batch to which the object data belongs. These also apply to FIGs. 8 and 9 described below.

As shown in FIG. 7, in the principal component analysis result of the mass spectrometry data MD, the biliary tract cancer patient samples (CCA, black) and the healthy subject samples (CTR, white) are located on a right side in a graph area, the pancreatic cancer patient samples (PDAC, vertical hatching) are located on a left side in the graph area, and both are largely separated to the left and right. In the present example, the biliary tract cancer patient samples and the healthy subject samples are samples collected first in the morning, while the pancreatic cancer patient samples are samples collected after anesthesia. Thus, it is considered that a variation caused by the differences in the sample collection conditions becomes a vector (the first principal component) of the largest variation. In this principal component analysis result, samples (rhombuses) of a batch 4 are located on an upper side in the graph area, the samples of the other batches are located on a lower side in the graph area, and both are largely separated vertically. Thus, it is considered that the second principal component is differences between the batches. The distribution of the biliary tract cancer patient samples (CCA, black) and the distribution of the healthy subject samples (CTR, white) completely overlap each other in the graph area, and the differences between the biliary tract cancer patient samples and the healthy subject samples are likely attributable to variation captured by the third principal component or beyond.

FIG. 8 is an explanatory chart showing the principal component analysis result of the mass spectrometry data MD narrowed down to the cancer-specific substances (features) as described above. As shown in FIG. 8, by selecting the "cancer-specific substances", it is possible to collect the substances unrelated to the influence of the anesthesia. Accordingly, in the principal component analysis result shown in FIG. 7, the distance between a group of the biliary tract cancer patient samples (CCA, black), the healthy subject samples (CTR, white) and the pancreatic cancer patient samples (PDAC, vertical hatching), which are separated in a direction of the first principal component (the variation due to the differences in the sample collection conditions), is considerably reduced. The biliary tract cancer patient samples (CCA, black) and the healthy subject samples (CTR, white) are separated from each other in a direction of the second principal component. However, in the principal component analysis result shown in FIG. 8, the biliary tract cancer patient samples (CCA, black) and the healthy subject samples (CTR, white) are not separated in the direction of the first principal component. Thus, it can be said that the first principal component is the variation caused by the differences in the sample collection conditions.

FIG. 9 is an explanatory chart showing the principal component analysis result of the comparison data CD generated based on the mass spectrometry data MD. In the principal component analysis result shown in FIG. 9, the biliary tract cancer patient samples (CCA, black) and the healthy subject samples (CTR, white) are clearly separated. More specifically, even in the case where the samples are classified as those of the biliary tract cancer patients when the value of the first principal component is positive, or the samples are classified as those of the healthy subjects when the value of the first principal component is negative, the diagnosis can be executed with high accuracy. That is, in this principal component analysis result, whether the object suffers from biliary tract cancer is a factor of the largest variation, and is in a state contributing to the diagnosis of biliary tract cancer. Since the healthy subject samples (CTR, white) and the pancreatic cancer patient samples (PDAC, vertical hatching) are largely separated vertically, it can be said that the second principal component is a variation in whether pancreatic cancer is present. The variation attributable to batch effects is primarily captured in the third principal component and beyond. The contribution rates of the first and second principal components became 22% and 18.9%, respectively, so that the total variance explained contributing to the diagnoses of biliary tract cancer and pancreatic cancer was boosted to 40.9%, while the contribution rate of the third principal component is reduced to 6%, and the variation caused by the differences between the batches was relatively reduced.

From the results of the principal component analyses shown in FIGs. 7 to 9, it can be said that, by using the diagnostic model MO created by the learning using the comparison data CD, it is possible to relatively reduce the influence of the variation in the value between the samples caused by the various factors and thus to achieve the diagnosis of the disease with the higher accuracy.

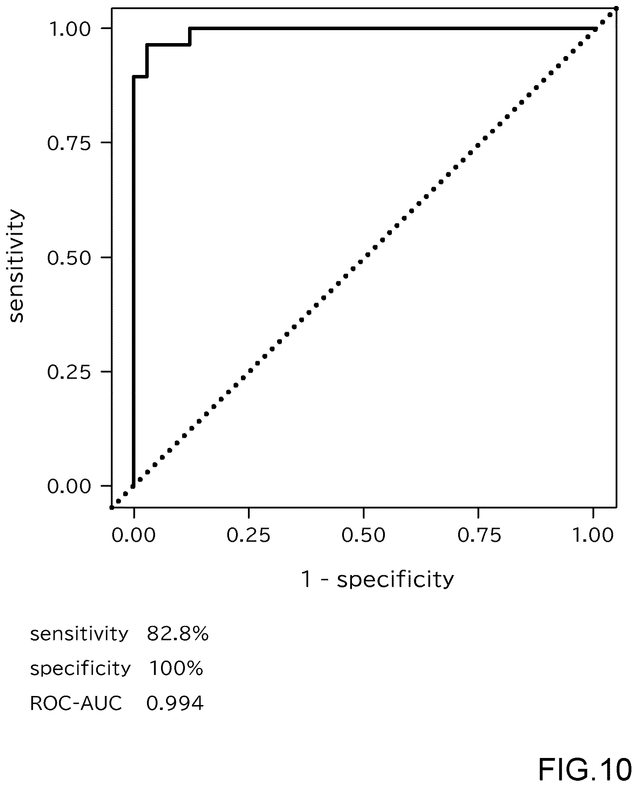

FIG. 10 is an explanatory chart showing accuracy of the diagnostic model MO in the present embodiment. FIG. 10 shows specificity and sensitivity when the diagnostic model MO was created by the learning using data submitted in 2021 and then verified by using data submitted in 2022 (i.e., a prospective validation setting). As shown in FIG. 10, the sensitivity: 82.8%, the specificity: 100%, and ROC-AUC: 0.994 are shown in the diagnosis of biliary tract cancer using the diagnostic model MO (the classification of whether the subject suffers from biliary tract cancer), and it can be said that the highly accurate diagnosis is achieved.

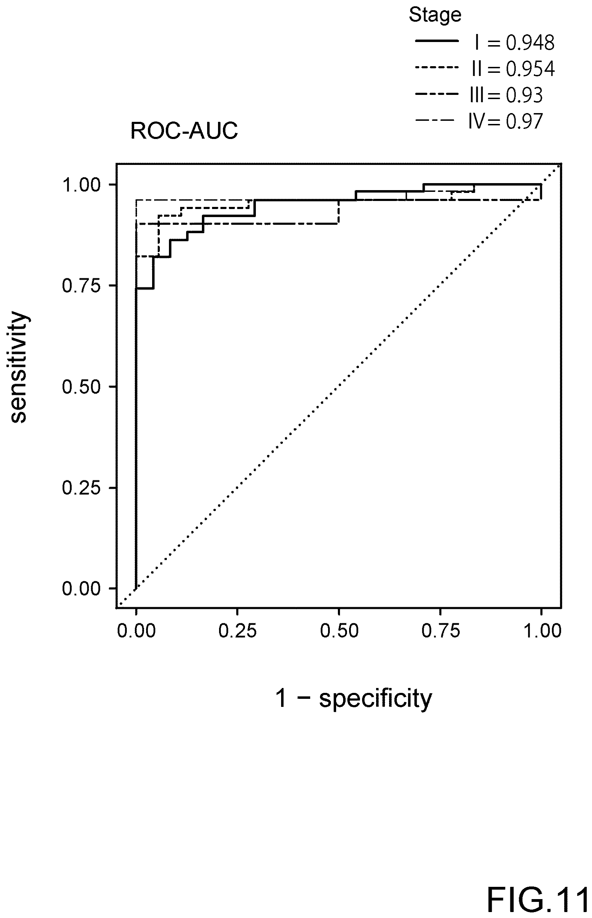

FIG. 11 is an explanatory chart showing the accuracy of the diagnostic model MO in the present embodiment. FIG. 11 shows the specificity and the sensitivity at each stage (stages 1 to 4) of pancreatic cancer when the diagnostic model MO in the present embodiment is applied to the data on pancreatic cancer. As shown in FIG. 11, in the diagnosis of pancreatic cancer by using the diagnostic model MO in the present embodiment, the accurate diagnosis is achieved not only at the stages 2 to 4, but also at the stage 1 at which the diagnosis is relatively difficult due to an early stage.

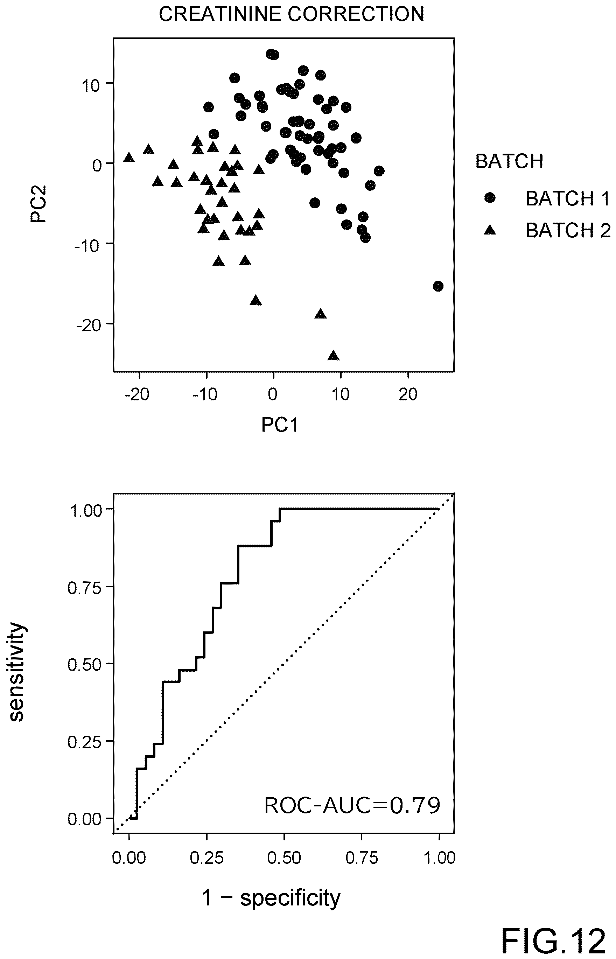

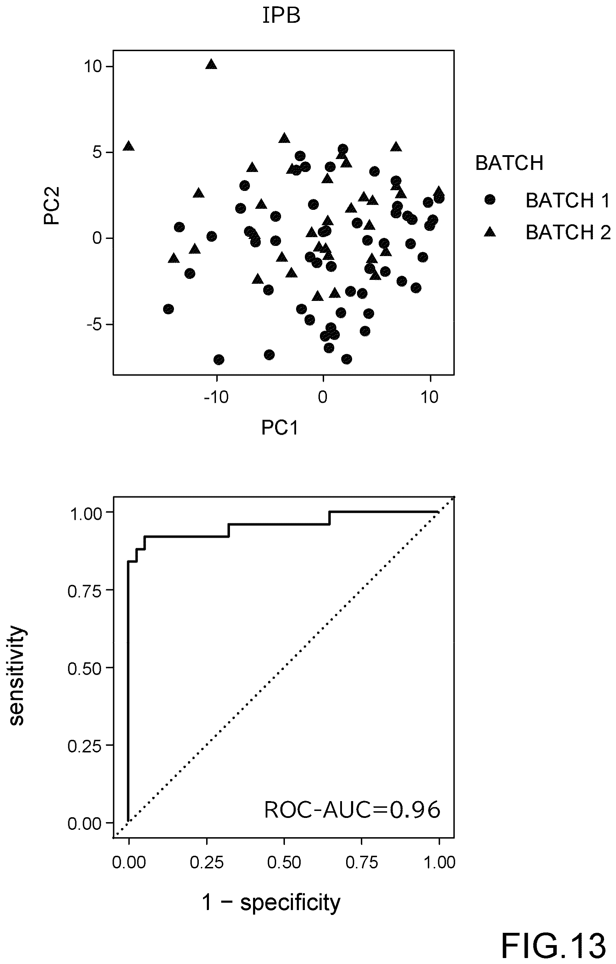

FIG. 12 and FIG. 13 are explanatory charts, each of which shows a comparison result between creatinine correction as a comparative example and the diagnostic model MO in the present embodiment. FIG. 12 and FIG. 13 each show the principal component analysis result and the classification accuracy when two data sets (the batch 1 and the batch 2) are classified by using the creatinine correction and the diagnostic model MO in the present embodiment. As shown in FIG. 12, in the creatinine correction, the batch 1 and the batch 2 are separated from each other, the variation caused by the differences between the batches is relatively large, and as a result, the classification accuracy is relatively low. As shown in FIG. 13, in the classification (IPB) using the diagnostic model MO in the present embodiment, the batch 1 and the batch 2 are not separated from each other, the variation caused by the differences between the batches is relatively small, and as a result, the classification accuracy is relatively high. In this way, according to the classification using the diagnostic model MO in the present embodiment, the accuracy of the classification can be improved in comparison with the classification using the creatinine correction.

### A-6. Effects of Present Embodiment:

As it has been described so far, the information processing device 100 in the present embodiment is the device for classifying the sample, and includes the model acquisition unit 114, the object data acquisition unit 115, and the classification execution unit 116. The model acquisition unit 114 acquires the diagnostic model MO as a learned model having the comparison data CD as the input and having the sample classification result as the output. In the mass spectrometry result for the plurality of the substances contained in the sample, for at least one substance group pair, which is the pair of the two substance groups each containing at least one substance, the comparison data CD contains the data indicating the magnitude relationship between the peak area of the substance belonging to one substance group and the peak area of the substance belonging to the other substance group. The object data acquisition unit 115 acquires the comparison data CD on the object sample. The classification execution unit 116 inputs the comparison data CD on the object sample to the diagnostic model MO, thereby classifying the object sample, and outputting the classification result.

As described above, in the information processing device 100 of the present embodiment, the object sample is classified by using the diagnostic model MO as the learned model which has the comparison data CD as the input and the sample classification result as the output. In the mass spectrometry result for the plurality of the substances contained in the sample, for at least one substance group pair, which is the pair of the two substance groups each containing at least one substance, the comparison data CD contains the data indicating the magnitude relationship between the peak area of the substance belonging to one substance group and the peak area of the substance belonging to the other substance group. Accordingly, by using the comparison data CD, it is possible to relatively reduce the influence of the variation in the value between the samples caused by the various factors and to improve the accuracy of the classification of the samples with reference to the mass spectrometry result. Since the comparison data CD is the data created by using the mass spectrometry results of the plurality of the substances contained in the sample, it is possible to improve the accuracy of the classification of the samples with reference to the mass spectrometry result in comparison with a configuration that only uses a mass spectrometry result of a specific substance.

In the information processing device 100 of the present embodiment, the comparison data CD may contain the data indicating the ratio of the peak area of the substance belonging to one substance group to the peak area of the substance belonging to the other substance group. With such a configuration, the substance group pair can be formed by any combination of the substances, without being limited to the pair of the substances whose peak areas in the sample are substantially the same and whose magnitude relationship is inverted depending on the belonging classification. As a result, a degree of freedom in setting of the substance group pair is increased. Thus, the further preferable comparison data CD can be acquired, and the accuracy of the classification of the sample with reference to the mass spectrometry result can be effectively improved.

In the information processing device 100 of the present embodiment, the comparison data CD may contain data indicating a ratio of the peak area of each of the substances belonging to one substance group to the average value of the peak areas of all the substances belonging to the other substance group. With such a configuration, the value of the comparison data CD is determined for each of the substances, and a reduction in an amount of the information can be suppressed. As a result, the comparison data CD having a larger amount of the information can be acquired, and the accuracy of the classification of the sample with reference to the mass spectrometry result can be effectively improved.

In the information processing device 100 of the present embodiment, the comparison data CD contains the data on the plurality of the substance group pairs. Thus, the comparison data CD having the larger amount of the information can be acquired, and the accuracy of the classification of the sample with reference to the mass spectrometry result can be effectively improved.

The information processing device 100 in the present embodiment further includes the pair setting unit 112 that sets at least one substance group pair based on the correlation between the plurality of the substances. Thus, it is possible to set the substance group pair that contributes to the further appropriate classification, and to effectively improve the accuracy of the classification of the sample with reference to the mass spectrometry result.

In the information processing device 100 of the present embodiment, the object sample may be a biological sample collected from a human. With such a configuration, it is possible to improve the accuracy of the classification of the biological sample with reference to the mass spectrometry result of the biological sample. The biological sample may be at least one of urine, bile, sweat, saliva, blood, cerebrospinal fluid (CSF), ascites, pleural fluid, pancreatic juice, gastric juice, and intestinal juice. With such a configuration, it is possible to improve the accuracy of the classification of the biological sample with reference to the mass spectrometry results of those biological samples.

In the information processing device 100 of the present embodiment, the classification execution unit 116 may execute the classification for the specific disease. With such a configuration, it is possible to improve the accuracy of the diagnosis of the specific disease with reference to the mass spectrometry result. The specific disease may be cancer. With such a configuration, it is possible to improve the accuracy of the diagnosis of cancer with reference to the mass spectrometry result.

### B. Modifications:

The technique disclosed in the present specification is not limited to the embodiments described above but can be modified in various forms without departing from the gist of the present specification, and, for example, the following modifications can be made.

The configuration of the information processing device 100 in the above-described embodiment constitutes merely one example, and various modifications can be made. The contents of the diagnostic model acquisition processing and the diagnostic processing in the above-described embodiment constitute merely one example, and various modifications can be made. For example, in the above-described embodiment, the information processing device 100 acquires the diagnostic model MO by generating the diagnostic model MO by itself. However, the information processing device 100 may acquire the diagnostic model MO generated by another device.

The configuration of the data and the machine learning algorithm used to create the diagnostic model MO in the above-described embodiment constitute merely one example, and various modifications can be made. For example, the comparison data CD used to create the diagnostic model MO may be created from the data for the single substance group pair, or may be created from the data for the plurality of the substance group pairs. In the example illustrated in FIG. 1, the comparison data CD for each of the substance group pairs may be a value acquired by dividing the peak area of the substance belonging to one substance group by the peak area of the substance belonging to the other substance group. In the example illustrated in FIG. 2, the comparison data CD for each of the substance group pairs may be a value acquired by dividing the average value of the peak areas of all the substances belonging to one substance group by the average value of the peak areas of all the substances belonging to the other substance group. In the example illustrated in FIG. 2, another representative value (for example, a median value) may be used instead of the average value. The mass spectrometry data MD or the comparison data CD does not have to be the data that specifies a specific substance name, and may be, for example, data that specifies a peak position (for example, an m/z value) of a mass spectrum acquired by the mass spectrometry.

The principal component analysis may be executed before the comparison data CD is input to the diagnostic model MO, and the first principal component, the second principal component, and the like may be used as the inputs to the diagnostic model MO.

In the above-described embodiment, the information processing for classifying whether the subject as the collection source of the sample suffers from biliary tract cancer is exemplified. However, the technique disclosed in the present specification is not limited to biliary tract cancer, and can be similarly applied to classification of whether the subject suffers from a specific disease. The technique disclosed in the present specification is not limited to the diagnosis, and can be similarly applied to a case where the sample is classified from a certain point of view.

In the above-described embodiment, at least one of the functional units included in the control unit 110 of the information processing device 100 may be included in another device instead of the control unit 110 of the information processing device 100. The diagnostic model acquisition processing and the diagnostic processing in the above-described embodiment are not necessarily executed by the single device, and may be separately executed by different devices. Steps of the diagnostic model acquisition processing in the above-described embodiment are not necessarily executed by the single device, and may be separately executed by different devices. Similarly, steps of the diagnostic processing in the above-described embodiment are not necessarily executed by the single device, and may be separately executed by different devices. In the above-described embodiment, a part of the configuration implemented by hardware may be replaced by software, and conversely, a part of the configuration implemented by the software may be replaced by the hardware.

The machine learning method may be a method using a single machine such as the random forest or the support vector machine, or may be an ensemble model (a stack model or a blend model) acquired by combining some of these machines.

The result of the mass spectrometry is not limited to the peak area of each of the substances, and may be any value of the peak intensity.

### DESCRIPTION OF REFERENCE NUMERALS

100: information processing device
110: control unit
111: original data acquisition unit
112: pair setting unit
113: training data acquisition unit
114: model acquisition unit
115: object data acquisition unit
116: classification execution unit
120: storage unit
130: display unit
140: operation input unit
150: interface unit
190: bus
CP: diagnostic program
LD: training data
MD: mass spectrometry data
MO: diagnostic model
RD: diagnostic result data

## Claims

1. An information processing device comprising:
a model acquisition unit that acquires a learned model taking comparison data as input and outputting a classification result of a sample, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group;
an object data acquisition unit that acquires the comparison data on an object sample; and
a classification execution unit that inputs the comparison data on the object sample to the learned model, thereby classifying the object sample, and outputting a result of the classification.

2. The information processing device according to claim 1, wherein
the comparison data contains data indicating a ratio of the peak intensity of the substance belonging to the one substance group to the peak intensity of the substance belonging to the other substance group.

3. The information processing device according to claim 2, wherein
the comparison data contains data indicating a ratio of the peak intensity of each of the substances belonging to the one substance group to an average value of the peak intensities of all the substances belonging to the other substance group.

4. The information processing device according to any one of claims 1 to 3, wherein
the comparison data contains data on a plurality of the substance group pairs.

5. The information processing device according to any one of claims 1 to 3 further comprising:
a pair setting unit that sets the at least one substance group pair based on correlation between the plurality of the substances.

6. The information processing device according to any one of claims 1 to 3, wherein
the sample is a biological sample derived from a human.

7. The information processing device according to claim 6, wherein
the biological sample is at least one of urine, bile, sweat, saliva, blood, cerebrospinal fluid (CSF), ascites, pleural fluid, pancreatic juice, gastric juice, and intestinal juice.

8. The information processing device according to claim 6, wherein
the classification execution unit executes the classification for a specific disease.

9. The information processing device according to claim 8, wherein
the specific disease is cancer.

10. An information processing device comprising:
a model acquisition unit that generates a learned model taking comparison data as input and outputting a classification result of a sample by performing training, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group.

11. An information processing method comprising:
a step of acquiring a learned model taking comparison data as input and outputting a classification result of a sample, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group;
a step of acquiring the comparison data on an object sample; and
a step of inputting the comparison data on the object sample to the learned model, thereby classifying the object sample, and outputting a result of the classification.

12. An information processing method comprising:
a step of generating a learned model taking comparison data as input and outputting a classification result of a sample by executing learning, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group.

13. A computer program causing a computer to execute:
processing to acquire a learned model taking comparison data as input and outputting a classification result of a sample, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group;
processing to acquire the comparison data on an object sample; and
processing to input the comparison data on the object sample to the learned model, thereby classifying the object sample, and outputting a result of the classification.

14. A computer program causing a computer to execute:
processing to generate a learned model taking comparison data as input and outputting a classification result of a sample by executing learning, in a mass spectrometry result of a plurality of substances contained in the sample, for at least one substance group pair, which is a pair of two substance groups each containing at least one substance, the comparison data containing data indicating a magnitude relationship between a peak intensity of the substance belonging to one of the substance groups and a peak intensity of the substance belonging to the other substance group.
